(19) Europäisches Patentamt
European Patent Office
Office européen des brevets

(11) Veröffentlichungsnummer: **0 135 722**
**B1**

(12) # EUROPÄISCHE PATENTSCHRIFT

(45) Veröffentlichungstag der Patentschrift: **16.09.87**

(51) Int. Cl.⁴: **C 07 K 7/00**, A 61 K 37/02

(21) Anmeldenummer: **84109101.0**

(22) Anmeldetag: **01.08.84**

(54) **Neue Peptide mit immunstimulierender Wirkung, Verfahren zu deren Herstellung und deren Verwendung.**

(30) Priorität: **03.08.83 DE 3327927**
**18.01.84 DE 3401545**

(43) Veröffentlichungstag der Anmeldung:
**03.04.85 Patentblatt 85/14**

(45) Bekanntmachung des Hinweises auf die Patenterteilung:
**16.09.87 Patentblatt 87/38**

(84) Benannte Vertragsstaaten:
**AT BE CH DE FR GB IT LI LU NL SE**

(56) Entgegenhaltungen:
**EP - A - 0 037 246**
**DE - A - 2 804 566**
**US - A - 4 298 523**

(73) Patentinhaber: **HOECHST AKTIENGESELLSCHAFT,**
**Postfach 80 03 20, D-6230 Frankfurt am Main 80 (DE)**

(72) Erfinder: **Kessler, Horst, Prof. Dr.,**
**Friedrich-Stoltze-Strasse 53, D-6231 Schwalbach (DE)**
Erfinder: **Kutscher, Bernhard, Am Erlenbruch 16,**
**D-6000 Frankfurt am Main (DE)**
Erfinder: **Obermeier, Rainer, Dr., Langenhainer Weg 14,**
**D-6234 Hattersheim am Main (DE)**
Erfinder: **Müllner, Hubert, Dr., Pestalozzistrasse 4,**
**D-6233 Kelkheim (Taunus) (DE)**

EP 0 135 722 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Aus Thymusextrakten wurden mehrere Peptide isoliert (z. B. Thymosin- und Thymopoietin II), die zur Differenzierung («Reifung») thymusabhängiger Lymphozyten (T-Zellen) beitragen. Auch eine Teilsequenz von Thymopoietin II, Arg-Lys-Asp-Val-Tyr, zeigt in den entsprechenden Testsystemen, z. B. dem Rosettentest, eine thymopoietinartige Wirkung (Science [1979] 204, 1309 bis 1310).

Für die Untersuchung über den Einfluss kleinerer Peptide auf Lymphozyten eignen sich neben dem genannten Rosettentest auch der PHA-Stimulationstest und der für B-Lymphozyten spezifische Plaque Forming Cell-Test (PFC-Test).

Im PHA-Stimulationstest (PHA-Phythämagglutinin) oder auch Lymphozytentransformationstest werden Rückschlüsse über die Anzahl der reifen, d. h. stimulierbaren Lymphozyten nicht über eine Untersuchung der Oberflächenantigene gewonnen, sondern durch den Funktionstest auf Stimulierbarkeit mit dem Pflanzenlektin PHA. Die Lymphozyten (T-Zellen) werden durch das Lektin, ähnlich wie durch bakterielle oder virale Antigene zu einer Blasentransformation angeregt. Diese führt entweder direkt oder durch Ausschüttung von Lymphokinen zu einer Proliferation. Der Einbau von radioaktiv markiertem Thymidin innerhalb einer bestimmten Zeit ist dann ein Mass für die Anzahl der stimulierten Zellen. Nur die reifen oder immunologisch potenten T-Zellen werden stimuliert. Es lässt sich daher der Einfluss einer Substanz für die Lymphozytenreifung mit diesem Test verfolgen. Allerdings muss die Stimulierung suboptimal sein, denn bei höheren Konzentrationen werden auch andere Subpopulationen von Lymphozyten stimuliert und der Effekt lässt sich nicht mehr beobachten. Die zu untersuchenden Peptide waren in verschiedenen Konzentrationen zum Kulturmedium gegeben worden. Sie sind während der gesamten Testdauer (bis zu 72 Stunden) präsent.

Im PFC-Test werden Zellkulturen aus frischpräparierten Milzen von Mäusen (2×107 Milzzellen pro ml) in RPMI 1640 und 30 µl foetalem Kälber-Serum (FKS) pro ml Zellkultur angelegt. Die in vitro Immunisierung erfolgt mit 5×107 Schaferythrozyten/ml. Die Test-Zellkulturen werden täglich mit der entsprechenden Dosis der Testsubstanz inkubiert.

Nach 5tägiger Laufzeit werden die Zellen abzentrifugiert, mit dem Medium RPMI 1640 gewaschen und der direkte PFC-Test durchgeführt. Dazu werden die Zellen zusammen mit einer 10%igen Schafserythrozyten-Suspension in einer Agaroselösung gemischt und auf einer ebenen Fläche ausgegossen. In der entstandenen Gelschicht geben die stimulierten Lymphozyten während der anschliessenden Inkubation Antikörper ab, die in die Umgebung diffundieren und sich an die hier befindlichen Schafserythrocyten heften. Nach der Zugabe von Meerschweinchen-Komplement lysieren die roten Blutzellen. Es entstehen helle, mit blossem Auge erkennbare runde Flecken in dem rötlich-braunen Gel, nämlich die Hämolysehöfe der Plaques. Im Zentrum solcher Hämolysehöfe befindet sich eine antikörperproduzierende Zelle. Die Anzahl lymphoider Zellen, die spezifische Immunglobuline bilden, kann folglich mit den ermittelten Plaque-Zahlen gleichgesetzt werden.

Es wurden nun neuartige cyclische Peptide gefunden, die überraschenderweise die bekannte Wirkung des linearen Peptides Arg-Lys-Asp-Val-Tyr übertreffen.

Die Erfindung betrifft Cyclopeptide der Formel I

$$\overline{\lfloor A-B-C-U-D \rfloor} \qquad (I)$$

in welcher

A Lys, D-Lys, Arg oder D-Arg,
B Lys, D-Lys, Arg oder D-Arg,
C Asp, D-Asp, Glu oder D-Glu,
U Val oder D-Val und
D Tyr, D-Tyr, Trp, D-Trp, Phe oder D-Phe bedeuten,

wobei 1 bis 4 der Reste A, B, C, U und D in der L-Konfiguration, die übrigen in der D-Konfiguration vorliegen, sowie deren physiologisch verträglichen Salze.

Bevorzugt sind solche Cyclopeptide der Formel I, in welcher U Val bedeutet und weiterhin solche, worin in Formel I

A D-Lys, oder Arg,
B Lys, D-Lys oder Arg,
C Asp oder Glu,
U D-Val und
D Tyr, Trp oder D-Phe bedeuten.

Die Erfindung betrifft ferner ein Verfahren zur Herstellung dieser Cyclopeptide, das dadurch gekennzeichnet ist, dass man lineare Peptidderivate der Formel II

$$\overline{\lfloor W-a-X-a-Y-a-U-a-D-a \rfloor} \qquad (II)$$

in welcher

W Lys ($R^1$), D-Lys ($R^1$), Arg ($R^2$) oder D-Arg ($R^2$),
X Lys ($R^1$), D-Lys ($R^1$), Arg ($R^2$) oder D-Arg ($R^2$) und
Y Asp ($R^3$), D-Asp ($R^3$), Glu ($R^3$) oder D-Glu ($R^3$) bedeuten und
U und D wie oben definiert sind, wobei
$R^1$ für eine Urethanschutzgruppe,
$R^2$ für eine Schutzgruppe der Guanidinofunktion und
$R^3$ für eine Esterschutzgruppe stehen, und einer der Reste a zusammen C-therminales OH + N-terminales H (a = –OH H–) zweier Aminosäurereste und die restlichen vier der Reste a jeweils Peptidbindungen bedeuten, cyclisiert, von den erhaltenen Verbindungen der Formel II, in welcher W, X, Y, U, D, $R^1$, $R^2$ und $R^3$ wie oben definiert sind, alle fünf Reste a für Peptidbindungen stehen und worin 1 bis 4 der Reste W, X, Y, U und D in der L-Konfiguration, die übrigen in der D-Konfiguration vorliegen, anschliessend die Schutzgruppen in an sich bekannter Weise entfernt und die erhaltenen Cyclopeptide der Formel I gegebenenfalls in ihre physiologisch verträglichen Salze überführt.

Unter den linearen Cyclisierungsvorstufen der Formel II werden Peptide der nachstehenden Formeln III bis VII verstanden,

H – W – X – Y – U – D – OH (III)
H – D – W – X – Y – U – OH (IV)
H – U – D – W – X – Y – OH (V)
H – Y – U – D – W – X – OH (VI)
H – X – Y – U – D – W – OH (VII)

in welchen W, X, Y, U, D, $R^1$, $R^2$ und $R^3$ wie oben definiert sind.

Die Cyclisierung der Peptide der Formeln II bis VI erfolgt vorzugsweise in Gegenwart von Kupplungsreagenzien wie DCC oder EDCI unter Zusatz von DMAP.

Urethanschutzgruppen der ε-Aminofunktion des Lysins sind beispielsweise Fmoc, Fcboc, Z, Boc, Ddz, Bpoc, Z-($NO_2$), Pyoc, Dobz, Moc, Mboc, Iboc, Adoc, Adpoc, Msc oder Pioc; bevorzugt sind Z oder Boc. Die Entfernung dieser Aminoschutzgruppen erfolgt mit Säuren, Basen oder reduktiv (vgl. Kontakte Merck 3/79, S. 14 ff.).

Schutzgruppen der Guanidinogruppe des Arginins sind z.B. $NO_2$, Tosyl, Boc, Z, Mesitylen-2-sulfonyl (Mts) u.ä. Die Abspaltung kann hydrolytisch oder hydrogenolytisch erfolgen (vgl. Kontakte Merck 1/80, S. 23, 30).

Die COOH-Seitenfunktionen von Asp und Glu werden als Alkylester, vorzugsweise Methyl-, Ethyl- oder tert.-Butylester oder als Benzylester oder modifizierter Benzylester (p-$NO_2$, p-Cl, p-Br u.a.) blockiert. Die Deblockierung erfolgt durch alkalische oder saure Verseifung oder Hydrierung (vgl. Kontakte Merck 3/79, S. 14, 20). Auch der durch Umsetzung mit Metallkatalysatoren leicht zu entfernende Allylester kommt in Frage (vgl. Angew. Chem. 96, 49 [1984]).

Die Erfindung betrifft weiterhin Peptide der Formel II

$$\lfloor W - a - X - a - Y - a - U - a - D - a \rfloor \quad (II)$$

in welcher W, X, Y, U, D, $R^1$, $R^2$ und $R^3$ die oben definierten Bedeutungen haben und

a) alle fünf Reste a für Peptidbindungen stehen oder

b) einer der Reste a zusammen C-terminales OH+N-terminales, (a = –OH H–) zweier Aminosäurereste W und X, X und Y, Y und U, U, und D, oder D und W und die restlichen vier der Reste a jeweils Peptidbindungen bedeuten.

Die linearen Peptide der Formel II (ein Rest a = –OH H–) können sowohl klassisch als auch mittels Festphasensynthese dargestellt werden.

Bei der klassischen Peptidsynthese der obigen linearen Peptide kann ein Aufbau durch Fragmente (konvergent) der rationellste Weg sein. Man erhält dabei ein lineares, geschütztes Pentapeptid, aus dem die linearen Peptide der Formeln III–VII durch Abspaltung der C- und N-terminalen Schutzgruppen erhalten werden. Im ersten Beispiel besitzt Trp die günstige N-terminale Position und wird somit einer minimalen Zahl von Schutzgruppenabspaltungen ausgesetzt, die bei Trp oft zu Nebenreaktionen führen. Ebenso könnte Tyr in dieser Position ungeschützt eingesetzt werden, da die minimale Anzahl von Kupplungsschritten bzw. von Schutzgruppenabspaltungen eine Reaktion der phenolischen OH-Gruppe unwahrscheinlich macht.

Die Auswahl der Schutzgruppen und die Synthesestrategie wird von der Art und Konfiguration der Aminosäuren sowie der Art der Kupplungs- und der Cyclisierungsbedingungen bestimmt.

Die erfindungsgemässen Peptide der Formel I enthalten einige mehrfunktionelle Aminosäuren, die einen erhöhten Aufwand der Schutzgruppentechnik (orthogonale Schutzgruppentaktik) erfordern. Bei der klassischen Peptidsynthese (durch Fragmentkondensation) benötigt man deshalb drei selektiv abspaltbare Schutzgruppen zum Aufbau der linearen Vorläufer der Formeln III–VII. Alle mehrfunktionellen Aminosäuren werden orthogonal geschützt eingesetzt, d.h. mit einer permanenten Schutzgruppe in die Seitenkette und einer dazu selektiv abspaltbaren C- oder N-terminalen Schutzgruppe zum Aufbau der Peptidkette. Die Aminosäuren ohne Seitenkettenfunktionalität werden C- oder N-terminal geschützt eingesetzt. Die entsprechenden Aminosäurederivate werden unter Verwendung eines Kupplungsreagenzes wie PPA oder DCC verknüpft. Nach dem selektiven Abspalten einer der C- oder N-terminalen Schutzgruppe des Dipeptidfragments werden die fehlenden C- oder N-terminal geschützten Aminosäurederivate oder in gleicher Weise hergestellte Fragmente ankondensiert, bis das gewünschte, voll geschützte lineare Pentapeptid hergestellt ist. Abspaltung der terminalen Schutzgruppen in möglichst einem Schritt, liefert dann die linearen Vorläufer III–VII.

Die C- und N-terminalen Schutzgruppen werden so gewählt, dass zur Herstellung der linearen, entschützten Pentapeptide der Formeln III–VII aus dem voll geschützten Peptid nur ein Abspaltungsschritt nötig ist. Eine solche Kombination stellen z.B. die Boc-Schutzgruppe und der tert.-Butylester dar, welche beide in einem Schritt z.B. durch Trifluoressigsäure abgespalten werden können.

Da ein globales Entschützen der Seitenkettenfunktionen nach der Cyclisierung günstig erscheint, werden dort vorzugsweise Schutzgruppen mit der gleichen Labilität gegenüber speziellen Abspaltungsreagenzien eingesetzt. So können z.B. die ε-Z-Schutzgruppe für Lysin, der β-Benzylester für Asp und ω-$NO_2$-Gruppe am Arg in einem Schritt hydrogenolytisch mit Hilfe eines Hydrierkatalysators (z.B. Pd/Aktivkohle) abgespalten werden.

Zur Cyclisierung der linearen Peptide nach Formel III–VII wurde eine neuartige Cyclisierungsmethode erarbeitet, da andere Cyclisierungsmethoden nur unbefriedigende Mengen der erfindungsgemässen Pentapeptide nach Formel I ergaben. Bei dem neuen Verfahren werden die linearen Vorläufer nach Formeln III–VII in aprotischen Lösungsmitteln wie DMF, $CH_2Cl_2$, $CHCl_3$, THF, Dioxan oder deren Gemischen gelöst.

Bei Salzen der linearen Verbindungen der Formeln III–VII werden diese durch Zugabe eines Äquivalents Base neutralisiert. Als Cyclisierungsreagenzien dienen z.B. DCC oder EDCI (ca. 20 Äquivalente) in Kombination mit ca. 10 Äquivalenten des Acylierungskatalysators DMAP. Beide Substanzen werden bei –15 °C zur Reaktionslösung gegeben und diese 6 Tage zwischen −2 ° und +25 °C belassen. Das Rohprodukt kann dann z. B. durch Gelchromatographie o. ä. gereinigt werden.

Nach Cyclisierung werden von den erhaltenen Peptiden der Formel II (alle Reste a bedeuten Peptidbindungen) die restlichen Schutzgruppen abgespalten.

Bei der Cyclisierung der linearen Peptide der Formeln III–VII wird in den Fällen, in denen die N- und die C-terminale Aminosäure dieser linearen Vorstufen die gleiche absolute Konfiguration aufweisen, eine Konfigurationsumkehr an der C-terminalen Aminosäure stattfinden.

Schema:

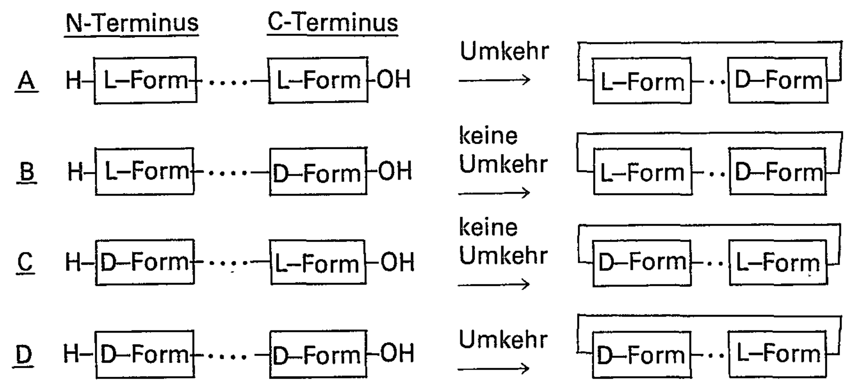


Die nachstehenden Beispiele veranschaulichen dieses Syntheseprinzip anhand einiger ausgewählter Verbindungen.

Tabelle 1

| | Edukte | Produkte der DCC (EDCI)/DMAP-Cyclisierung |
|---|---|---|
| a) | H-Tyr-Arg($NO_2$)-Lys(Z)-Glu(OBzl)-Val-OH | cyclo-[-Tyr-Arg($NO_2$)-Lys(Z)-Glu(OBzl)-D-Val-] |
| b) | H-Tyr-Arg($NO_2$)-Lys(Z)-Asp(OBzl)-Val-OH | cyclo-[-Tyr-Arg($NO_2$)-Lys(Z)-Asp(OBzl)-D-Val-] |
| c) | H-Trp-Arg($NO_2$)-Lys(Z)-Glu(OBzl)-Val-OH | cyclo-[-Tyr-Arg($NO_2$)-Lys(Z)-Glu(OBzl)-D-Val-] |
| d) | H-Trp-Arg($NO_2$)-Lys(Z)-Asp(OBzl)-Val-OH | cyclo-[-Tyr-Arg($NO_2$)-Lys(Z)-Asp(OBZl)-D-Val-] |
| e) | H-Tyr-Arg($NO_2$)-D-Lys(Z)-Glu(OBzl)-Val-OH | cyclo-[-Tyr-Arg($NO_2$)-D-Lys(Z)-Glu(OBzl)-D-Val-] |
| f) | H-Tyr-Arg($NO_2$)-D-Lys(Z)-Glu(OBzl)-Val-OH | cyclo-[-Tyr-Arg($NO_2$)-D-Lys(Z)-Glu(OBzl)-D-Val-] |
| g) | H-Arg-($NO_2$)-Lys(Z)-Asp(OBzl)-Val-Tyr-OH | cyclo-[-Arg($NO_2$)-Lys(Z)-Asp(OBzl)-Val-D-Tyr-] |
| h) | H-Tyr-Arg($NO_2$)-Lys(Z)-Glu(OBzl)-D-Val-OH | cyclo-[-Tyr-Arg($NO_2$)-Lys(Z)-Glu(OBzl)-D-Val-] |
| i) | H-Tyr-Arg($NO_2$)-Lys(Z)-Asp(OBzl)-D-Val-OH | cyclo-[-Tyr-Arg($NO_2$)-Lys(Z)-Asp(OBzl)-D-Val-] |
| j) | H-D-Lys(Z)-Arg($NO_2$)-Glu(OBzl)-Val-Tyr-OH | cyclo-[-D-Lys($NO_2$)-Arg($NO_2$)-Glu(OBzl)-Val-Tyr] |
| k) | H-D-Lys(Z)-Arg($NO_2$)-Asp(OBzl)-Val-Tyr-OH | cyclo-[-D-Lys($NO_2$)-Arg($NO_2$)-Asp(OBzl)-Val-Tyr] |
| l) | H-D-Phe-Arg($NO_2$)-Lys(Z)-Glu(OBzl)-Val-OH | cyclo-[-D-Phe-Arg($NO_2$)-Lys(Z)-Glu(OBzl)-Val-] |

Wie man aus obigem Schema erkennen kann, weisen die Cyclisierungsprodukte, unabhängig davon, ob von A oder B ausgegangen wurde, die gleichen Konfigurationen an der Cyclisierungsstelle auf. Entsprechendes gilt für C und D.

Die oben unter a)–g) aufgeführten Cyclopeptide enthalten nach der Cyclisierung mit DCC(EDCI)/DMAP die im linearen Vorläufer C-terminal stehende Aminosäure als D-Aminosäure. Die linearen Edukte enthalten hierbei keine oder eine in der Mitte der Sequenz stehende D-Aminosäure.

Die Chiralität der C-terminalen Aminosäure wurde in den Cyclopeptiden unter a) und f) durch chirale Aminosäureanalyse als D-Aminosäure bestimmt. Die NMR-Spektren lassen keine Zweifel daran, dass aus diesen Daten auch auf die Cyclopeptide unter b)–e) geschlossen werden kann. Das Cyclopeptid unter g) hat, lässt man die Konfiguration ausser acht, die gleiche Sequenz wie das unter b), die andere Cyclisierungsstelle führt jedoch zu einem anderen stereoisomeren Produkt.

Einige der obigen Cyclopeptide enthalten schon in linearen Edukten in der C- oder in der N-terminalen Position D-Aminosäuren. Hier zeigt sich keine Racemisierung bzw. Inversion der C-terminalen Aminosäuren. Die Cyclopeptide unter h) und i) entsprechen den aus all-L-Aminosäure-Vorläufern hergestellten Cyclopeptiden unter a) und b). Cyclopeptid Pos. j) zeigt kein D-Tyr in der chiralen Aminosäureanalyse. Cyclopeptid Pos. l) zeigt im $^1$H-NMR-Spektrum deutliche Ähnlichkeit mit Cyclopeptid Pos. g). Die Cyclopeptide unter a), j) und l) werden auch durch die Azid-Cyclisierung (racemisierungsarm!) erhalten.

Unter physiologisch verträglichen Salzen der erfindungsgemässen Cyclopeptide der Formel I werden vor allem Säureadditionssalze mit anorganischen Säuren wie HCl, $HNO_3$, $H_2SO_4$, $H_3PO_4$ oder organischen Säuren wie Weinsäure, Citronensäure, Maleinsäure oder Basenadditionssalze mit Alkali- oder Erdalkalihydroxiden oder physiologisch verträglichen Aminen verstanden.

Die erfindungsgemässen Cyclopeptide wurden im Plaqueforming Cell Assay (PFC-Test) und im Phythämagglutinin-Stimulationstest (PHA-Test)

auf ihre lymphozyten-stimulierende Wirkung getestet.

Ein möglicher Abbau der Peptide durch Serum oder Lymphozytenproteasen wird bei diesen Untersuchungen nicht berücksichtigt.

Die erfindungsgemässen Verbindungen können zur Behandlung von Immundefizienzen, viralen und fungoiden sowie chronisch bakteriellen Infekten, Autoimmunkrankheiten, wie zur Therapie von Erkrankungen dienen, die durch Zellen mit immunologisch relevanten Veränderungen der Zellmembranmerkmale (z. B. Tumorzellen) verursacht werden.

Die Erfindung betrifft weiterhin die Verwendung der genannten Peptide ganz allgemein zur Beeinflussung der Reifung von T-Lymphocyten sowie Mittel, die diese Peptide als Wirkstoff enthalten.

Die Anwendung der erfindungsgemässen Peptide kann intravenös, subcutan oder intranasal erfolgen. Beim normalgewichtigen Erwachsenen liegt die Dosierung bei parenteraler Gabe bei 0,001–10 mg, bei intranasaler Gabe 0,01–10 mg je Einzeldosis. Sie kann in schweren Fällen auch erhöht werden, da toxische Eigenschaften bisher nicht beobachtet wurden. Auch eine Herabsetzung der Dosis ist möglich.

Die erfindungsgemässen Verbindungen können intranasal oder parenteral in entsprechender pharmazeutischer Zubereitung verabreicht werden. Für eine intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wässrige, alkoholische oder ölige Suspensionen oder wässrige, alkoholische oder ölige Lösungen. Als ölige Trägerstoffe oder Lösungsmittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsmittler, Emulgatoren oder weitere Hilfsstoffe in Lösung, Suspension oder Emulsion gebracht.

Als Lösungsmittel für die neuen aktiven Verbindungen und die entsprechenden physiologisch verträglichen Salze kommen z. B. in Frage: Wasser, physiologische Kochsalzlösungen oder Alkohole, z. B. Ethanol, Propandiol oder Glycerin, daneben auch Zuckerlösungen wie Glucose- oder Mannitlösungen, oder auch eine Mischung aus den verschiedenen genannten Lösungsmitteln.

Nachfolgende Testergebnisse (Tabelle 2) geben eine Auswahl über die biologische Aktivität der erfindungsgemässen Peptide im PFC- und PHA-Test.

Tabelle 2
Dosierung 100 ng/Substanz/l ml Zellkultur

| | PFC/$10^6$ Zellen | PHA Stimulationsindex 10 µg PHA/1 ml 72 h |
|---|---|---|
| Kontrolle (unbehandelt) | 221±30 | 1,00 |
| Arg-Lys-Asp-Val-Tyr | 298±43 | 1,05 |
| cyclo-(Arg-Lys-Asp-D-Val-Tyr) | 401±64 | 1,69 |
| cyclo-(Arg-Lys-Glu-D-Val-Tyr) | 687±73 | 1,92 |
| cyclo-(D-Lys-Arg-Glu-Val-Tyr) | 317±41 | 1,15 |
| cyclo-(Arg-Lys-Glu-D-Val-Trp) | 369±54 | 1,25 |
| cyclo-(Arg-Lys-Asp-D-Val-Trp) | 312±58 | – |
| cyclo-(D-Lys-Arg-Asp-Val-Tyr) | 341±43 | – |
| cyclo-(Arg-Lys-Glu-Val-D-Phe) | 250±31 | – |

Zur weiteren Erläuterung sollen die folgenden Synthesebeispiele dienen, ohne dass die Erfindung auf diese beschränkt wäre:

Beispiel 1
Darstellung von cyclo-(Arg-Lys-Asp-D-Val-Tyr) ·2HOAc (1) nach Syntheseschema I

a) BOC-Arg($NO_2$)-Lys(Z)-OMe (3)

8,25 g (25 mmol) H-Lys(Z)-OMe·HCl und 7,98 g (25 mmol) Boc-Arg($NO_2$)-OH werden in 100 ml $CH_2Cl_2$ suspendiert, auf –15 °C abgekühlt und mit 12,94 ml (0,118 mol) NMM versetzt. Unter $N_2$-Atmosphäre werden dann 22 g (50,5 mmol) PPA-Lösung (50% in $CH_2Cl_2$, 0,88 g/mmol) zugetropft. Nach langsamem Erwärmen wird 2 Tage bei Raumtemperatur gerührt. Das Lösungsmittel wird am Rotationsverdampfer abgezogen und der Rückstand zwischen 300 ml EE und 150 ml ges. $NaHCO_3$-Lösung verteilt. Die organische Phase wird zweimal mit je 80 ml ges. $NaHCO_3$-Lösung, dreimal mit je 80 ml 5%iger Citronensäurelösung und nochmals mit 80 ml ges. NaCl-Lösung gewaschen. Die vereinigten EE-Phasen werden über $MgSO_4$ getrocknet, das Lösungsmittel abgezogen und der Rückstand zu einem festen Schaum getrocknet.

Ausbeute 13,8 g (93%); Rf A 0,31; B 0,54; C 0,71; $[\alpha]^{20}_{D} = -7,44°$ (c = 1, abs. MeOH)
$C_{26}H_{21}B_7O_9$ (595,35)
ber.: C 52,44 H 6,94 N 16,47
gef.: C 52,33 H 6,82 N 16,20

b) Ddz-Asp(OBzl)-Val-OBut (4)

Die Peptidkupplung erfolgt wie bei a) beschrieben nach der PPA-Methode.

Ansatz: 13,4 g (30 mmol) Ddz-Asp(OBzl)-OH und 10,36 g (30 mmol) H-Val-OBut·TsOH.

Das Produkt fällt als Öl an.

Ausbeute: 16 g (88%); Rf A 0,68; B 0,69; C 0,75

c) H-Arg($NO_2$)-Lys(Z)-OMe·HCl (5)

Zur Abspaltung der Boc-Schutzgruppe werden 9,53 g (16 mmol) 3 mit 160 ml 2N HCl/MeOH 2 h bei Raumtemperatur gerührt. Danach wird die Lösung eingeengt, mehrfach in MeOH aufgenommen und wieder eingedampft. Das Produkt wird aus EtOH/Diethylether umkristallisiert.

Ausbeute 8,2 g (97%)
Schmp. 165–168 °C; Rf A 0,0; B 0,41; C 0,34; $[\alpha]_D^{20} = +9,61°$
(c = 1, abs. MeOH)
$C_{21}H_{32}N_7O_7Cl$ (529,74)
ber.: C 47,61 H 6,08 N 18,51
gef.: C 47,90 H 6,30 N 18,55

d) H-Asp(OBzl)-Val-OBut·TFA (6)

Zur Abspaltung der Schutzgruppe werden 12 g (20 mmol) 4 in 130 ml $CH_2Cl_2$ gelöst und 4,6 ml (60 mmol) TFA zugegeben (3,5% TFA/$CH_2Cl_2$).

Die Lösung wird 30 min bei Raumtemperatur gerührt und das Lösungsmittel abgezogen. Der Rückstand wird aus Diethylether/Petrolether umkristallisiert.

Ausbeute: 9,15 g (93%);
Schmp. 113–116 °C; Rf A 0,25 ; B 0,68; C 0,70; $[\alpha]_D^{20} = -3,56°$ (c = 1, abs. MeOH)

e) Boc-Tyr-Arg($NO_2$)-Lys(Z)-OMe (7)

Die Peptidkupplung erfolgt wie unter a) beschrieben nach der PPA-Methode.

Ansatz: 5,55 g (12 mmol) Boc-Tyr-OH·DCHA und 6,35 g (12 mmol) 5.

Das Produkt wird aus EtOH/Diethylether umkristallisiert.

Ausbeute: 8,65 g (95%);
Schmp. 128–130 °C; Rf A 0,15; B 0,80; C 0,85; $[\alpha]_D^{20} = -6,8°$ (c = 1, abs. MeOH)

f) Boc-Tyr-Arg($NO_2$)-Lys(Z)-OH (8)

Zur Verseifung des Methylesters werden 8,5 g (11 mmol) 7 in 33 ml MeOH gelöst und mit 26 ml 1N NaOH versetzt. Die wässrige Lösung wird mit 2N HCl auf pH 2 angesäuert und dreimal mit je 80 ml EE extrahiert. Die wässrige Phase wird mit NaCl gesättigt und nochmals dreimal mit je 80 ml EE extrahiert. Die vereinigten organischen Phasen werden über $Na_2SO_4$ getrocknet. Das Lösungsmittel wird entfernt und der Rückstand zu einem festen Schaum getrocknet.

Ausbeute: 7,4 g (90%); Rf A 0,19; B 0,57; C 0,48; $[\alpha]_D^{20} = -5,66°$ (c = 1, abs. MeOH)

g) Boc-Tyr-Arg($NO_2$)-Lys(Z)-Asp(OBzl)-Val-OBut (9)

Die Peptidkupplung erfolgt wie bei a) beschrieben nach der PPA-Methode.

Ansatz: 4,46 g (6 mmol) 8 und 3,25 g (6,6 mmol) 6. Das nach der Aufarbeitung erhaltene Rohprodukt wird durch Gelchromatographie an Sephadex LH 20 mit DMF als Laufmittel gereinigt. Das Produkt wird aus EtOH/Diethylether umkristallisiert.

Ausbeute: 3,6 g (54%);
Schmp. 108–111 °C; Rf A 0,0; B 0,91; C 0,84; $[\alpha]_D^{20} = -19,4°$ (c = 1, abs. MeOH)
$C_{54}H_{76}N_{10}O_{15}$ (1105,18)
ber.: C 58,69 H 6,92 N 12,67
gef.: C 58,44 H 6,78 N 12,54

h) H-Tyr-Arg($NO_2$)-Lys(Z)-Asp(OBzl)-Val-OH·TFA (10)

Zur gleichzeitigen Abspaltung der Boc- und tert.-Butylester-Schutzgruppen werden 2,65 g (2,4 mmol) 9 mit 7,4 ml (96 mmol) TFA versetzt und 10 min bei Raumtemperatur unter $N_2$-Atmosphäre gerührt. Die Lösung wird mit 50 ml $CH_2Cl_2$ aufgenommen und eingedampft. Das Produkt wird dann aus MeOH/Diethylether umkristallisiert und über KOH getrocknet.

Ausbeute: 2,39 g (95%);
Schmp. 158–160 °C; Rf A 0,0; B 0,63; C 0,57

i) cyclo-(Tyr-Arg($NO_2$)-Lys(Z)-Asp(OBzl)-D-Val) (11)

2,1 g (2,0 mmol) 11 werden in einer Mischung von 300 ml DMF und 1,7 l $CH_2Cl_2$ gelöst (= 1 mmol Peptid pro 1 l Lösungsmittel) und auf –15 °C abgekühlt. Es werden 0,18 ml (2 mmol) NMM und 2,44 g (20 mmol) DMAP zugegeben und zur Lösung 3,83 (20 mmol) EDCI, gelöst in 25 ml DMF, zugetropft. Die Reaktionslösung wird drei Tage bei –2/5 °C stehengelassen, dann auf –15 °C abgekühlt und mit 3,83 g (20 mmol) EDCI nachaktiviert. Nach zwei Tagen bei –2/+5 °C und einem Tag bei Raumtemperatur wird die Reaktionslösung im Vakuum eingedampft. Der nach dem Einengen verbliebene ölige Rückstand wird mit 300 ml $H_2O$ versetzt und der ausgefallene Niederschlag abgesaugt. Das erhaltene Rohprodukt wird durch Gelchromatographie an Sephadex LH 20 mit DMF als Laufmittel gereinigt und das erhaltene Produkt aus DMF/MeOH/Diethylether umkristallisiert.

Ausbeute: 558 mg (30%); Schmp. 238–242 °C (Zers.); Rf A 0,0; B 0,88; C 0,92; $[\alpha]_D^{20} = -45,58°$ (c = 0,6 DMF)
$C_{45}H_{58}N_{10}O_{12}$ (930,95)
ber.: C 58,05 H 6,27 N 15,05
gef.: C 57,85 H 6,07 N 15,08

j) cyclo-(Tyr-Arg-Lys-Asp-D-Val)·2HOAc ≡ cyclo-(Arg-Lys-Asp-D-Val-Tyr)·2 HOAc (1)

Zur Abspaltung der Seitenkettenschutzgruppen werden 93 mg (0,1 mmol) 11 in 2 ml MeOH und 5 ml HOAc gelöst, mit 80 mg Katalysator (10% Pd/Aktivkohle) versetzt und 6 h bei Raumtemperatur hydriert. Der Katalysator wird abfiltriert, die Lösung eingeengt und der Rückstand über KOH getrocknet.

Ausbeute: 63 mg (81%); Schmp. 205 °C (Zers.); Rf A 0,0; B 0,22; C 0,0; D 0,42;

Aminosäureanalyse: Asp 1,0; D-Val 1,01, Tys 0,81; Lys 1,00; Arg 0,97

Gehalt: 97%

Beispiel 2

Darstellung von cyclo-(Arg-Lys-Glu-D-Val-Tyr)·2 HOAc (2) nach Syntheseschema I

Die gewünschte Verbindung wird nach den gleichen experimentellen Verfahren hergestellt wie für Beispiel 1 beschrieben. Verbindungen, die zur Darstellung von 2 benötigt werden und schon in Beispiel 1 beschrieben wurden, werden mit der dort verwandten Ziffer bezeichnet.

a) Ddz-Glu(OBzl)-Val-OBut (12)

Die Peptidkupplung wird wie in Beispiel 1 unter a) beschrieben nach der PPA-Methode ausgeführt.

Ansatz: 15,1 g (32 mmol) Ddz-Glu(OBzl)-OH und 11,4 g (32 mmol) H-Val-OBut·TsOH. Das Produkt fällt als Öl an.

Ausbeute: 18,3 g (93%); Rf A 0,70; B 0,65; C 0,78.

b) H-Glu(OBzl)-Val-OBut·TFA (13)

Abspaltung der Ddz-Schutzgruppe wie in Beispiel 1 unter d) beschrieben.

Ansatz: 17,8 g (29 mmol) 12, 6,7 ml (87 mmol) TFA und 188 mmol $CH_2Cl_2$.

Das Produkt wird aus Diethylether/Petrolether umkristallisiert.

Ausbeute: 12,7 g (86%); Schmp. 132–136 °C; Rf A 0,2; B 0,65; C 0,70; $[\alpha]_D^{20} = -0,23°$ (c = 1, MeOH)

$C_{23}H_{33}N_2O_7F_3$ (506,49)

ber.: C 54,54 H 6,56 N 5,58

gef.: C 54,82 H 6,40 N 5,53

c) Boc-Tyr-Arg($NO_2$)-Lys(Z)-Gly(OBzl)-Val-OBut (14)

Die Peptidkupplung und die Reinigung des Rohprodukts erfolgen wie in Beispiel 1 unter g) beschrieben.

Ansatz: 5,916 g (18 mmol) 8 und 5,06 g (10 mmol) 13.

Das Produkt wird aus MeOH/EE umkristallisiert.

Ausbeute: 6,1 g (68%); Schmp. 149–153 °C; Rf A 0,1; B 0,85; C 0,91; $[\alpha]_D^{20} = -20,84°$ (c = 1, MeOH)

$C_{55}H_{78}N_{10}O_{15}$ (1119,2)

ber.: C 59,02 H 7,02 N 12,52

gef.: C 58,78 Z 6,94 N 12,29

d) H-Tyr-Arg($NO_2$)-Lys(Z)-Glu(OBzl)-Val-OH·TFA (15)

Ausführung wie bei Beispiel 1 unter h) beschrieben.

Ansatz: 2,75 g (2,45 mmol) 14 und 7,6 ml (98 mmol) TFA.

Ausbeute 2,4 g (92%); Rf A 0,00; B 0,63; C 0,58.

e) cyclo-(Tyr-Arg($NO_2$)-Lys(Z)-Glu(OBzl)-D-Val) (16)

Die Cyclisierung erfolgt wie in Beispiel 1 unter i) beschrieben.

Ansatz: 2,4 g (2,2 mmol) 15, 0,2 ml (2,2 mmol) NMM; 2,68 g (22 mmol) DMAP und 8,4 g (44 mmol) EDCI.

Das nach der Gelchromatographie erhaltene Produkt wird aus DMF/MeOH/Diethylether umkristallisiert.

Ausbeute: 686 mg (33%); Schmp. 234–238 °C (Zers.); Rf A 0,00; B 0,80; C 0,86; $[\alpha]_D^{20} = -58,46°$ (c = 0,26, DMF);

$C_{46}H_{60}N_{10}O_{12}$ (944,98)

ber.: C 54,47 H 6,39 N 14,82

gef.: C 57,91 H 6,52 N 14,94

FAB-MS: 945

f) cyclo-(Tyr-Arg-Lys-Glu-D-Val)·2 HOAc ≡ cyclo-(Arg-Lys-Glu-D-Val-Tyr)·2 HOAc (2)

Abspaltung der Seitenkettenschutzgruppen wie in Beispiel 1 unter j) beschrieben.

Ansatz: 94 mg (0,1 mmol) 16;

Ausbeute: 65 mg (82%); Schmp. 140–142 °C (Zers.); Rf A 0,00; B 0,22; C 0,00; D 0,44.

Aminosäureanalyse: Glu 0,98; D-Val 1,01; Tyr 0,84; Lys 0,98; Arg 1,00

Gehalt: 92%

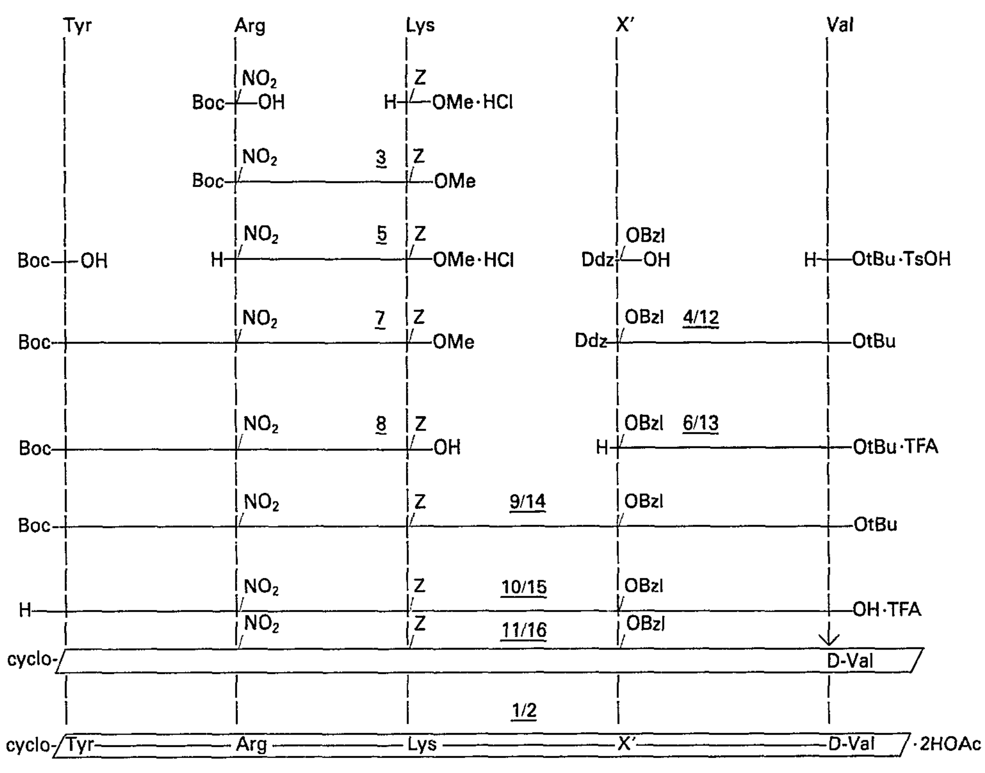

X' = Asp/Glu

Syntheseschema I

Abkürzungen für Schutzgruppen und Reagenzien

| | |
|---|---|
| Boc | = tert.-Butyloxycarbonyl |
| Bzl | = Benzyl- |
| DCC | = Dicyclohexylcarbodiimid |
| DCHA | = Dicyclohexylamin |
| | = α,α'-Dimethyl-3,5-dimethoxybenzyloxycarbonyl |
| DMAP | = 4-Dimethylaminopyridin |
| DMF | = N,N-Dimethylformamid |
| EDCl | = 1-Ethyl-3-(3-dimethylaminopropyl)carbodiimidhydrochlorid |
| EE | = Essigester (Ethylacetat) |
| EtOH | = Ethanol |
| HOAc | = Essigsäure |
| MeOH | = Methanol |
| NMM | = N-Methylmorpholin |
| OMe | = Methoxy |
| OBut | = tert.-Butoxy |
| PPA | = n-Propylphosphonsäureanhydrid |
| TsOH | = p-Toluolsulfonsäure |
| Z | = Carbobenzoxycarbonyl |

Abkürzungen im Text

| | |
|---|---|
| abs. | = absolut |
| ges. | = gesättigt |
| Schmp. | = Schmelzpunkt |

Laufmittel für die Dünnschichtchromatographie
A Chloroform/Methanol/Eisessig (95:5:3)
B n-Butanol/Eisessig/Wasser (3:1:1)
C Essigester/n-Butanol/Pyridin/Wasser (20:10:3:5)
D Methanol/Eisessig (1:1)

**Patentansprüche für die Vertragsstaaten: BE, CH, DE, FR, GB, IT, LI, LU, NL, SE**

1. Cyclopeptide der Formel I

$$\overline{\text{A} - \text{B} - \text{C} - \text{U} - \text{D}} \qquad (I)$$

in welcher
A Lys, D-Lys, Arg oder D-Arg,
B Lys, D-Lys, Arg oder D-Arg,
C Asp, D-Asp, Glu oder D-Glu,
U Val oder D-Val und
D Tyr, D-Tyr, Trp, D-Trp, Phe oder D-Phe
bedeuten, wobei 1 bis 4 der Reste A, B, C, U und D in der L-Konfiguration, die übrigen in der D-Konfiguration vorliegen, sowie deren physiologisch verträglichen Salze.

2. Cyclopeptide gemäss Anspruch 1, dadurch gekennzeichnet, dass
A D-Lys, oder Arg,
B Lys, D-Lys oder Arg,
C Asp oder Glu,
U D-Val und
D Tyr, Trp oder D-Phe bedeuten.

3. Cyclopeptide gemäss Anspruch 1, dadurch gekennzeichnet, dass U Val bedeutet.

4. Verfahren zur Herstellung von Cyclopeptiden der Formel I gemäss einem der Ansprüche 1 bis 3, dadurch gekennzeichnet, dass man lineare Peptidderivate der Formel II,

$$\overline{\text{W} - \text{a} - \text{X} - \text{a} - \text{Y} - \text{a} - \text{U} - \text{a} - \text{D} - \text{a}} \qquad (II)$$

in welcher
W Lys ($R^1$), D-Lys ($R^1$), Arg ($R^2$) oder D-Arg ($R^2$),
X Lys ($R^1$), D-Lys ($R^1$), Arg ($R^2$) oder D-Arg ($R^2$) und
Y Asp ($R^3$), D-Asp ($R^3$), Glu ($R^3$) oder D-Glu ($R^3$) bedeuten und
U und D wie in Anspruch 1 definiert sind, wobei
$R^1$ für eine Urethanschutzgruppe,
$R^2$ für eine Schutzgruppe der Guanidinofunktion und
$R^3$ für eine Esterschutzgruppe stehen, und
einer der Reste a zusammen C-terminales OH+N-terminales H (a= –OH H–) zweier Aminosäurereste und die restlichen vier der Reste a jeweils Peptidbindungen bedeuten, cyclisiert, von den erhaltenen Verbindungen der Formel II, in welcher W, X, Y, U, D, $R^1$, $R^2$ und $R^3$ wie oben definiert sind, alle fünf Reste a für Peptidbindungen stehen und worin 1 bis 4 der Reste W, X, Y, U und D in der L-Konfiguration, die übrigen in der D-Konfiguration vorliegen, anschliessend die Schutzgruppen in an sich bekannter Weise entfernt und die erhaltenen Cyclopeptide der Formel I gegebenenfalls in ihre physiologisch verträglichen Salze überführt.

5. Peptide der Formel II, in welcher W, X, Y, U, D, $R^1$, $R^2$ und $R^3$ wie im Anspruch 4 definiert sind und
a) alle fünf Reste a für Peptidbindungen stehen oder
b) einer der Reste a zusammen C-terminales OH+N-terminales H (a = –OH+H–) zweier Aminosäurereste und die restlichen vier der Reste a jeweils Peptidbindungen bedeuten.

6. Verfahren zur Herstellung von Peptiden der Formel II gemäss Anspruch 5, dadurch gekennzeichnet, dass man diese in an sich bekannter Weise durch Fragmentkondensation herstellt und gegebenenfalls cyclisiert.

7. Cyclopeptide der Formel I gemäss einem der Ansprüche 1 bis 3 zur Verwendung als Heilmittel.

8. Heilmittel, enthaltend ein Cyclopeptid der Formel I gemäss einem der Ansprüche 1 bis 3.

9. Verfahren zur Herstellung eines Heilmittels gemäss Anspruch 8, dadurch gekennzeichnet, dass man ein Cyclopeptid der Formel I in eine geeignete Darreichungsform bringt.

**Patentansprüche für den Vertragsstaat: AT**

1. Verfahren zur Herstellung von Cyclopeptiden der Formel I

$$\overline{A-B-C-U-D} \quad (I)$$

in welcher

A Lys, D-Lys, Arg oder D-Arg,
B Lys, D-Lys, Arg oder D-Arg,
C Asp, D-Asp, Glu oder D-Glu,
U Val oder D-Val und
D Tyr, D-Tyr, Trp, D-Trp, Phe oder D-Phe

bedeuten, wobei 1 bis 4 der Reste A, B, C, U und D in der L-Konfiguration, die übrigen in der D-Konfiguration vorliegen, sowie deren physiologisch verträglichen Salzen, dadurch gekennzeichnet, dass man lineare Peptidderivate der Formel II

$$\overline{W-a-X-a-Y-a-U-a-D-a} \quad (II)$$

in welcher

W Lys ($R^1$), D-Lys ($R^1$), Arg ($R^2$) oder D-Arg ($R^2$),
X Lys ($R^1$), D-Lys ($R^1$), Arg ($R^2$) oder D-Arg ($R^2$) und
Y Asp ($R^3$), D-Asp ($R^3$), Glu ($R^3$) oder D-Glu ($R^3$)

bedeuten und

U und D wie oben definiert sind, wobei
$R^1$ für eine Urethanschutzgruppe,
$R^2$ für eine Schutzgruppe oder Guanidinofunktion und
$R^3$ für eine Esterschutzgruppe stehen, und

einer der Reste a zusammen C-terminales OH+N-terminales H (a = –OH H–) zweier Aminosäurereste und die restlichen vier der Reste a jeweils Peptidverbindungen bedeuten, cyclisiert, von den erhaltenen Verbindungen der Formel II, in welcher W, X, Y, U, D, $R^1$, $R^2$ und $R^3$ wie oben definiert sind, alle fünf Reste a für Peptidverbindungen stehen und worin 1 bis 4 der Reste W, X, Y, U und D in der L-Konfiguration, die übrigen in der D-Konfiguration vorliegen, anschliessend die Schutzgruppen in an sich bekannter Weise entfernt und die erhaltenen Cyclopeptide der Formel I gegebenenfalls in ihre physiologisch verträglichen Salze überführt.

2. Verfahren gemäss Anspruch 1 zur Herstellung von Cyclopeptiden der Formel I, in welcher

A D-Lys, oder Arg,
B Lys, D-Lys oder Arg,
C Asp oder Glu,
U D-Val und
D Tyr, Trp oder D-Phe bedeuten.

3. Verfahren gemäss Anspruch 1, zur Herstellung von Cyclopeptiden der Formel I, in welcher U Val bedeutet.

4. Verfahren zur Herstellung von Peptiden der Formel II, in welcher W, X, Y, U, D, $R^1$, $R^2$ und $R^3$ wie im Anspruch 3 definiert sind und

a) alle fünf Reste a für Peptidbindungen stehen oder

b) einer der Reste a zusammen C-terminales OH+N-terminales H (a = –OH+H–) zweier Aminosäurereste und die restlichen vier der Reste a jeweils Peptidbindungen bedeuten, dadurch gekennzeichnet, dass man diese in an sich bekannter Weise durch Fragmentkondensation herstellt und gegebenenfalls cyclisiert.

5. Verfahren gemäss einem der Ansprüche 1 bis 3 zur Herstellung von Cyclopeptiden der Formel I zur Verwendung als Heilmittel.

6. Verfahren zur Herstellung eines Heilmittels, enthaltend ein Cyclopeptid gemäss einem der Ansprüche 1 bis 3, dadurch gekennzeichnet, dass man diese in eine geeignete Darreichungsform bringt.

**Revendications pour les Etats contractants: BE, FR, IT, LI, CH, DE, GB, LU, NL, SE**

1. Cyclopeptides de formule I

$$\overline{A-B-C-U-D} \quad (I)$$

dans laquelle

A représente Lys, D-Lys, Arg ou D-Arg,
B représente Lys, D-Lys, Arg ou D-Arg,
C représente Asp, D-Asp, Glu ou D-Glu,
U représente Val ou D-Val et
D représente Tyr, D-Tyr, Trp, D-Trp, Phe ou D-Phe, auquel cas de 1 à 4 des groupes A, B, C, U et D sont dans la configuration L, et les autres sont dans la configuration D; ainsi que leurs sels physiologiquement acceptables.

2. Cyclopeptides selon la revendication 1, caractérisés en ce que

A représente D-Lys, ou Arg,
B représente Lys, D-Lys ou Arg,
C représente Asp ou Glu,
U représente D-Val ou D-Val et
D représente Tyr, Trp ou D-Phe.

3. Cyclopeptides selon la revendication 1, caractérisés en ce que U représente Val.

4. Procédé pour la préparation de cyclopeptides de formule I selon l'une quelconque des revendications 1 à 3, caractérisé en ce qu'on cyclise des dérivés peptidiques linéaires de formule II

$$\overline{W-a-X-a-Y-a-U-a-D-a} \quad (II)$$

dans laquelle

W représente Lys ($R^1$), D-Lys ($R^1$), Arg ($R^2$) ou D-Arg ($R^2$),
X représente Lys ($R^1$), D-Lys ($R^1$), Arg ($R^2$) ou D-Arg ($R^2$), et
Y représente Asp ($R^3$), D-Asp ($R^3$), Glu ($R^3$) ou D-Glu ($R^3$) et

U et D sont tels que définis dans la revendication 1, auquel cas

$R^1$ représente un groupe uréthane-protecteur,

$R^2$ représente un groupe protecteur pour la fonction guanidino et

$R^3$ représente un groupe ester-protecteur et l'un des restes a représente simultanément un groupe OH C-terminal et un atome H N-terminal (a = –OH H–) de deux groupes acide aminé et les quatre autres restes a représentent chacun des liaisons peptide, parmi les composés de formule II obtenus, dans lesquels W, X, Y, U, D, $R^1$, $R^2$ et $R^3$ sont tels que définis ci-dessus, les cinq restes a représentent tous des liaisons peptide et de 1 à 4 des restes W, X, Y, U et D sont dans la configuration L et les autres dans la configuration D,

puis on élimine les groupes protecteurs de manière connue en soi et on convertit éventuellement les cyclopeptides obtenus de formule I obtenus en leurs sels physiologiquement acceptables.

5. Peptides de formule II, dans lesquels W, X, Y, U, D, $R^1$, $R^2$ et $R^3$ sont tels que définis dans la revendication 4 et

a) tous les cinq restes a représentent des liaisons peptide ou

b) l'un des restes a représente simultanément un groupe OH C-terminal et un atome H N-terminal (a = –OH H–) de deux groupes amino-acide et les quatre autres restes a représentent chacun des liaisons peptide.

6. Procédé pour la préparation de peptides de formule II selon la revendication 5, caractérisé en ce qu'on prépare ceux-ci d'une manière connue en soi par condensation de fragments et qu'on cyclise éventuellement.

7. Cyclopeptides de formule I selon l'une quelconque des revendications 1 à 3, utilisables comme médicaments.

8. Médicament contenant un cyclopeptide de formule I selon l'une quelconque des revendications 1 à 3.

9. Procédé pour la préparation d'un médicament selon la revendication 8, caractérisé en ce qu'on met un cyclopeptide de formule I sous une forme d'administration appropriée.

**Revendications pour l'Etat contractant: AT**

1. Procédé pour la préparation de cyclopeptides de formule I

$$\ulcorner A - B - C - U - D \urcorner \quad (I)$$

dans laquelle

A représente Lys, D-Lys, Arg ou D-Arg,

B représente Lys, D-Lys, Arg ou D-Arg,

C représente Asp, D-Asp, Glu ou D-Glou,

U représente Val ou D-Val et

D représente Tyr, D-Tyr, Trp, D-Trp, Phe ou D-Pheauquel cas de 1 à 4 des groupes A, B, C, U et D sont dans la configuration L, les autres sont dans la configuration D, ainsi que de leurs sels physiologiquement acceptables, caractérisé en ce qu'on cyclise des dérivés peptidiques linéaires de Formule II

$$\ulcorner W - a - X - a - Y - a - U - a - D - a \urcorner \quad (II)$$

dans laquelle

W représente Lys ($R^1$), D-Lys ($R^1$), Arg ($R^2$) ou D-Arg ($R^2$),

X représente Lys ($R^1$), D-Lys ($R^1$), Arg ($R^2$) ou D-Arg ($R^2$), et

Y représente Asp ($R^3$), D-Asp ($R^3$), Glu ($R^3$) ou D-Glu ($R^3$) et

U et D sont tels que définis ci-dessus, auquel cas

$R^1$ représente un groupe uréthanne-protecteur,

$R^2$ représente un groupe protecteur pour la fonction guanidino et

$R^3$ représente un groupe ester-protecteur; et l'un des restes a représente simultanément un groupe OH C-terminal et un atome H N-terminal (a = –OH H–) de deux groupes acide aminé et les quatre autres restes a représentent chacun des liaisons peptide, parmi les composés de formule II obtenus, dans lesquels W, X, Y, U, D, $R^1$, $R^2$ et $R^3$ sont tels que définis ci-dessus, les cinq restes a représentent tous des liaisons peptide et auquel cas de 1 à 4 des restes W, X, Y, U et D sont dans la configuration L, et les autres dans la configuration D,

puis on élimine les groupes protecteurs d'une manière connue en soi et éventuellement on convertit les cyclopeptides de formule I obtenus en leurs sels physiologiquement acceptables.

2. Procédé selon la revendication 1 pour la préparation de cyclopeptides de formule I, dans laquelle

A représente D-Lys, ou Arg,

B représente Lys, D-Lys ou Arg,

C représente Asp ou Glu,

U représente D-Val et

D représente Tyr, Trp ou D-Phe.

3. Procédé selon la revendication 1 pour la préparation de cyclopeptides de formule I dans laquelle U représente Val.

4. Procédé pour la préparation de peptides de formule II, dans laquelle W, X, Y, U, D, $R^1$, $R^2$ et $R^3$ sont tels que définis dans la revendication 3 et

a) les cinq restes a représentent tous les liaisons peptide ou

b) l'un des restes a représente simultanément un groupe OH C-terminal et un atome H N-terminal (a = –OH H–) de deux groupes acide aminé et les quatre autres restes a représentent chacun des liaisons peptide, caractérisé en ce qu'on prépare ceux-ci d'une manière connue en soi par condensation de fragments et qu'on cyclise éventuellement.

5. Procedé selon l'une quelconque des revendications 1 à 3 pour la préparation de cyclopeptides de formule 1, utilisables comme médicaments.

6. Procédé pour la préparation d'un médicament contenant un cyclopeptide selon l'une quelconque des revendications 1 à 3, caractérisé en ce qu'on met ce dernier sous une forme d'administration appropriée.

**Claims for the contracting states: BE, CH, DE, FR, GB, IT, LI, LU, NL, SE**

1. A cyclopeptide of the formula I

$$\ulcorner A-B-C-U-D \urcorner \quad (I)$$

in which
A denotes Lys, D-Lys, Arg or D-Arg,
B denotes Lys, D-Lys, Arg or D-Arg,
C denotes Asp, D-Asp, Glu or D-Glu,
U denotes Val or D-Val and
D denotes Tyr, D-Tyr, Trp, D-Trp, Phe or D-Phe,
1 to 4 of the radicals A, B, C, U and D being in the L-configuration and the remainder being in the D-configuration, and a physiologically acceptable salt thereof.

2. A cyclopeptide as claimed in claim 1, in which
A denotes D-Lys or Arg,
B denotes Lys, D-Lys or Arg,
C denotes Asp or Glu,
U denotes D-Val and
D denotes Tyr, Trp or D-Phe.

3. A cyclopeptide as claimed in claim 1, wherein U denotes Val.

4. A process for the preparation of a cyclopeptide of the formula I as claimed in any one of claims 1 to 3, which comprises cyclizing a linear peptide derivative of the formula II

$$\ulcorner W-a-X-a-Y-a-U-a-D-a \urcorner \quad (II)$$

in which
W denotes Lys ($R^1$), D-Lys ($R^1$), Arg ($R^2$) or D-Arg ($R^2$),
X denotes Lys ($R^1$), D-Lys ($R^1$), Arg ($R^2$) or D-Arg ($R^2$) and
Y denotes Asp ($R^3$), D-Asp ($R^3$), Glu ($R^3$) or D-Glu ($R^3$) and
U and D are as defined in claim 1,
and in which
$R^1$ represents a urethane-protective group,
$R^2$ represents a protective group for the guanidino function and
$R^3$ represents an ester-protective group, and
one of the radicals a denotes the C-terminal OH together with the N-terminal H (a = –OH H–) of two aminoacid radicals and the remaining four radicals a each denote peptide bonds, and then removing, in a manner which is known per se, the protective groups from the resulting compounds of the formula II, in which W, X, Y, U, D, $R^1$, $R^2$ and $R^3$ are as defined above, all the five radicals a represent peptide bonds and 1 to 4 of the radicals W, X, Y, U and D are in the L-configuration and the remainder are in the D-configuration, and, if appropriate, the resulting cyclopeptide of the formula I is converted into its physiologically acceptable salt.

5. A peptide of the formula II, in which W, X, Y, U, D, $R^1$, $R^2$ and $R^3$ are as defined in claim 4 and
a) all five radicals a represent peptide bonds or
b) one of the radicals a denotes a C-terminal OH together with an N-terminal H (a = –OH H–) of two aminoacid radicals and the remaining four radicals a each denote peptide bonds.

6. A process for the preparation of a peptide of the formula II as claimed in claim 5, which comprises preparing this peptide, and if appropriate cyclizing the product, in a manner which is known per se by fragment condensation.

7. A cyclopeptide of the formula I as claimed in any one of claims 1 to 3 for use as a healing agent.

8. A healing agent containing a cyclopeptide of the formula I as claimed in any one of claims 1 to 3.

9. A process for the preparation of a healing agent as claimed in claim 8, which comprises bringing a cyclopeptide of the formula I into a suitable form for administration.

**Claims for the contracting state: Austria**

1. A process for the preparation of a cyclopeptide of the formula I

$$\ulcorner A-B-C-U-D \urcorner \quad (I)$$

in which
A denotes Lys, D-Lys, Arg or D-Arg,
B denotes Lys, D-Lys, Arg or D-Arg,
C denotes Asp, D-Asp, Glu or D-Glu,
U denotes Val or D-Val and
D denotes Tyr, D-Tyr, Trp, D-Trp, Phe or D-Phe,
1 to 4 of the radicals A, B, C, U and D being in the L-configuration and the remainder being in the D-configuration, and a physiologically acceptable salt thereof, which comprises cyclizing a linear peptide derivative of the formula II

$$\ulcorner W-a-X-a-Y-a-U-a-D-a \urcorner \quad (II)$$

in which
W denotes Lys ($R^1$), D-Lys ($R^1$), Arg ($R^2$) or D-Arg ($R^2$),
X denotes Lys ($R^1$), D-Lys ($R^1$), Arg ($R^2$) or D-Arg ($R^2$) and
Y denotes Asp ($R^3$), D-Asp ($R^3$), Glu ($R^3$) or D-Glu ($R^3$) and
U and D are as defined above
and in which
$R^1$ represents a urethane-protective group,
$R^2$ represents a protective group for the guanidino function and
$R^3$ represents an ester-protective group, and
one of the radicals a denotes the C-terminal OH together with the N-terminal H (a = –OH H–) of two aminoacid radicals and the remaining four radicals a each denote peptide bonds, and then removing, in a manner which is known per se, the protective groups from the resulting compounds of the formula II, in which W, X, Y, U, D, $R^1$, $R^2$ and $R^3$ are as defined above, all the five radicals a represent peptide bonds and 1 to 4 of the radicals W, X, Y, U and D are in the L-configuration and the remainder are in the D-configuration, and, if appropriate, the resulting cyclopeptide of the formula I is converted into its physiologically acceptable salt.

2. A process as claimed in claim 1 for the preparation of a cyclopeptide of the formula I, in which

A denotes D-Lys or Arg,
B denotes Lys, D-Lys or Arg,
C denotes Asp or Glu,
U denotes D-Val and
D denotes Tyr, Trp or D-Phe.

3. A process as claimed in claim 1 for the preparation of a cyclopeptide of the formula I, wherein U denotes Val.

4. A process for the preparation of a peptide of the formula II, in which W, X, Y, U, D, $R^1$, $R^2$ and $R^3$ are as defined in claim 3 and

a) all five radicals a represent peptide bonds or

b) one of the radicals a denotes a C-terminal OH together with an N-terminal H (a = –OH H–) of two aminoacid radicals and the remaining four radicals a each denote peptide bonds, which comprises preparing this peptide, and if appropriate cyclizing the product, in a manner which is known per se by fragment condensation.

5. A process as claimed in any one of claims 1 to 3 for the preparation of a cyclopeptide of the formula I for use as a healing agent.

6. A process for the preparation of a healing agent containing a cyclopeptide as claimed in any one of claims 1 to 3, which comprises bringing a cyclopeptide of the formula I into a suitable form for administration.